# EUROPEAN PATENT SPECIFICATION

(11) **EP 0 972 513 B1**
(45) Date of publication and mention of the grant of the patent: **02.05.2003**
(21) Application number: 98947774.0
(22) Date of filing: 07.10.1998
(51) Int. Cl.: A61K 9/20, A61K 47/36, A61K 47/46

(54) **PROCESS FOR PREPARING EMULSIFIED POWDER**
VERFAHREN ZUR HERSTELLUNG VON EMULGIERTEM PUDER
PROCEDE DE PREPARATION DE POUDRE EMULSIONNEE

(30) Priority: 07.10.1997 JP 27484297
(43) Date of publication of application: 19.01.2000
(73) Proprietor: Eisai Co., Ltd., Tokyo 112-8088 (JP)
(72) Inventor: KATO, Yoshiteru, Saitama 367-0043 (JP); SUZUKI, Toru, Tokyo 202-0004 (JP); ABE, Shinobu, Saitama 362-0042 (JP); TOKIZANE, Makoto, Saitama 330-0038 (JP)
(74) Representative: Gille Hrabal Struck Neidlein Prop Roos
(86) International application number: JP9804532
(87) International publication number: WO99017746

(56) References cited:
- WO-A1-90/07327
- JP-A- 3 240 723
- JP-A- 3 258 730
- JP-A- 52 015 812
- JP-A- 62 032 844
- US-A- 3 184 385
- DATABASE CHEMABS [Online] CHEMICAL ABSTRACTS SERVICE, COLUMBUS, OHIO, US; SHEKERDZHIISKI, R. ET AL: "Study of possible microencapsulation of vitamin E acetate" retrieved from STN Database accession no. 104:116022 XP002175973 & FARMATSIYA (SOFIA) (1985), 35(4), 23-8 ,

## Description

### TECHNICAL FIELD

This invention relates to a process for the preparation of emulsified powders of an oil-soluble substance. The emulsified powders prepared according to the invention are suitable for tableting, because the cells encapsulating oil particles are hardly disintegrated by compressing impact, leading to less oil exudation.

### BACKGROUND ART

US 3,184,385 discloses a vitamin E powder prepared from a modified starch, starch alumino succinate, in water via an emulsion of vitamin E oil.

Emulsified powders containing oil-soluble substances such as edible fats have been prepared by emulsifying and dispersing oil-soluble substances in water using an emulsifying agent such as gum arabic, a filler such as dextrin or the like and then drying the emulsion thus obtained by a conventional drying means such as spray drying, drum drying, belt drying, freeze drying or the like. It has been also carried out to emulsify oil-soluble substances using as an emulsifying agent synthetic surfactants such as glycerol fatty acid esters, polyglycerol fatty acid esters, sucrose fatty acid esters and the like.

The emulsified powders prepared using gum arabic have a tough coated film and are relatively suited for tableting, but the emulsified particles are difficult to be finely divided. Further, because gum arabic in an amount of about two or three times that of the oil-soluble substance is required, the oil-soluble substance has a limited content.

Although synthetic surfactants are excellent in emulsifying property, a coated film is so weak that the emulsified cells may be disintegrated during the drying step and oil exudation is observed over the surface of powders at a higher rate, and the surfactants are not suited for pulverizing an oil-soluble substance.

As an alternative to the emulsified powders, there have been proposed mononuclear capsules by microcapsulation using gelatin, but they have the drawbacks that the surface thereof may be converted to a hardened film by formalin treatment or the like, so that there is a high possibility of disintegration in the coated film portion and said portion is disintegrated in the course of tableting.

As is explained above, when tablets or the like are to be prepared using the emulsified powders of the oil-soluble substance as prepared according to the prior art, oil exudation may be frequently brought about by compressing the emulsified powders having a higher content of the oil-soluble substance, thus the amount thereof to be added is limited. In the case of the emulsified powders having a lower content of the oil-soluble substance, a larger amount of the emulsified powders should be used, but the amount thereof to be added has its limit in view of the composition of a tablet. Therefore, tablets having a higher content of the oil-soluble substance are presently not obtainable. When a large amount of the oil-soluble substance is to be ingested in the form of tablets as health food, tablets having a higher content of the oil-soluble substance are desired. Under these circumstances, there have been eagerly expected emulsified powders of an oil-soluble substance which do not induce any oil exudation in the course of tableting.

### DISCLOSURE OF INVENTION

It is an object of the present invention to provide emulsified powders containing an oil-soluble substance, wherein the cells containing oil particles are stable against impact and oil exudation is not brought about in the course of tableting.

We have made earnest studies to solve the above problems, and as a result, have found that less disintegrative emulsified powders can be obtained by emulsifying a core substance of an oil-soluble substance such as edible fats or the like with a chemically modified starch and a vegetable gum in water and drying the resulting emulsified dispersion using a conventional drying means, upon which the present invention has been completed.

Accordingly, the invention relates to a process for the preparation of emulsified powders which comprises emulsifying an oil-soluble substance, a chemically modified starch and a vegetable gum in water and then drying the emulsion.

The emulsified powders prepared according to the invention are an aggregate of a great number of particles (cells) in which a core substance of the oil-soluble substance is coated with the chemically modified starch or the like, and a particle diameter thereof is preferably 50-500 µ. The emulsified particles when emulsified in water have preferably a particle diameter of 2 µ or less.

The oil-soluble substance which may be used in the invention includes fat-soluble vitamins such as vitamin A, vitamin E, vitamin A acetate, vitamin E acetate and the like; unsaturated higher fatty acids such as DHA (docosahexaenoic acid), EPA (eicosapentaenoic acid) and the like; oil-soluble flavors such as orange oil, lemon oil, peppermint oil and the like.

The chemically modified starch as used in the invention refers to an ester of a starch with an organic acid having an emulsifying activity, which is referred to as "Food starch-modified" in the Gazette of the United States, CFR (FDA) 172.89 (d), 1995. Examples of said organic acid may include succinic acid, acetic acid, adipic acid or an alkyl or alkenyl derivative thereof such as octenyl succinate. As the chemically modified starch is particularly preferred octenyl succinate starch, which is a half ester of a starch and octenyl succinate (See U.S. Patent No. 3,971,852), or a salt thereof, whose examples may include "EMULSTAR 30A" (trade name, manufactured by MATSUTANI KAGAKU KOGYO CO., LTD.), "CAPSULE" (trade name, manufactured by National Starch & Chemical Nippon NSC LTD.)

The vegetable gum which may be used in the invention includes gum arabic, xanthan gum, guar gum and the like, as well as pullulan, pectin and the like.

In practicing the present process, to a mixture of 150-200 parts (parts by weight, which will be similarly applied hereinafter), 1-90 parts of the chemically modified starch and 1-50 parts of the vegetable gum was added 10-60 parts of the oil-soluble substance and then the mixture was stirred at a temperature of 10-60°C at a revolution number of 5000-15000 per minute for 5-20 minutes to prepare an emulsion of the oil-soluble substance. In order to reduce disintegration during tableting, it is desirable that an emulsified particle diameter may be preferably 2 µ or less. The emulsifier to be applied may be suitably a high-speed agitating emulsifier with a higher shear force such as "CLEARMIX" (trade name, manufactured by M TECHNIQUE) or a high pressure emulsifier such as a homogenizer, but it is not limited to said emulsifiers. The emulsion thus prepared may be dried as such by any suitable means such as spray drying, drum drying, belt drying, freeze drying or the like to prepare emulsified powders having excellent tableting performances. The emulsified powders obtained by the process of the invention have a good water-dispersion property and also a superior fluidizing property.

### EXAMPLES

The present invention will be more specifically illustrated by way of the following Examples.

### Example 1

In 150 g of water were dissolved 20 g of a chemically modified starch (manufactured by MATSUTANI KAGAKU KOGYO CO., LTD.; trade name of "EMULSTAR 30A"), 30 g of lactose and 20 g of gum arabic and the solution was sterilized by heating at 85-90°C for 15 minutes. This solution was cooled to 40°C or lower, 30 g of vitamin E was added and admixed and then emulsified by means of TK-HOMO-MIXER (manufactured by TOKUSHU KIKA KOGYO CO., LTD.). The emulsified particles were re-emulsified by means of "CLEARMIX" (trade name of an emulsifier manufactured by M TECHNIQUE) so as to provide finely divided particles. The emulsion thus prepared was spray-dried at an inlet temperature of 140°C and an exhaust temperature of 90°C using a spray drier (manufactured by OHKAWARA KAKOHKI CO., LTD.) to afford 90 g of emulsified powders of vitamin E (Product 1 of the invention).

### Example 2

In 150 g of deaerated water were dissolved 30 g of a chemically modified starch (manufactured by National Starch & Chemical Nippon NSC LTD.; trade name of "CAPSULE"), 14 g of lactose and 25 g of gum arabic and the solution was sterilized by heating at 85-90°C for 15 minutes. This solution was cooled to 40°C or lower, 30 g of DHA oil and 1 g of vitamin E were added and admixed and then emulsified by means of TK-HOMO-MIXER. The emulsified particles were re-emulsified by means of "CLEARMIX" (trade name of an emulsifier manufactured by M TECHNIQUE) so as to provide finely divided particles. The emulsion thus prepared was spray-dried at an inlet temperature of 140°C and an exhaust temperature of 90°C using a spray drier (manufactured by OHKAWARA KAKOHKI CO., LTD.) to afford 90 g of emulsified powders of DHA (Product 2 of the invention).

### Example 3

Following the same procedure as described in Example 2 except that 30 g of peppermint oil was used instead of 30 g of the DHA oil, there was obtained 90 g of emulsified powders of peppermint oil.

### Example 4 (Comparison)

In 150 g of water were dissolved 20 g of the chemically modified starch as used in Example 1 and 50 g of dextrin (DE 10) and the solution was sterilized and then 30 g of vitamin E was added. Then, the same procedure as described in Example 1 was repeated to afford 90 g of emulsified powders of vitamin E (Product 4 as comparison).

### Example 5 (Comparison)

In 150 g of deaerated water were dissolved 30 g of the chemically modified starch as used in Example 2 and 39 g of dextrin (DE 10) and the solution was sterilized. Then, 30 g of DHA oil and 1 g of vitamin E were added. The same procedure as described in Example 1 was repeated to afford 90 g of emulsified powders of DHA (Product 5 as comparison).

### Example 6 (Comparison)

In 15 kg of purified water was dissolved 5 kg of a chemically modified starch (manufactured by MATSUTANI KAGAKU KOGYO CO., LTD.; "EMULSTAR 30A"). To the solution was added 5 kg of vitamin E acetate (manufactured by EISAI CO., LTD.) and then the mixture was emulsified by means of the TK-HOMO-MIXER and a pressure emulsifier. The emulsion was spray-dried at an inlet temperature of 160°C and an exhaust temperature of 80°C by means of a spray drier (manufactured by OHKAWARA KAKOHKI CO., LTD.) to prepare emulsified powders of vitamin E. To 990 g of the emulsified vitamin E powders thus prepared was added 10 g of hydrous silicon dioxide (manufactured by FUJI SILYSIA CHEMICAL LTD.; "SILYSIA 355") as a fluidizing agent and admixed to afford emulsified powders of vitamin E (Product 6 as comparison).

### Reference Example 1

Following the same procedure as described in Example 4 except that an equal amount of gum arabic was used instead of the chemically modified starch, there was obtained 90 g of emulsified powders of vitamin E (Reference Product 1).

### Reference Example 2

Following the same procedure as described in Example 5 except that an equal amount of gum arabic was used instead of the chemically modified starch, there was obtained 90 g of emulsified powders of DHA (Reference Product 2).

### Comparative Example 1

Tablets were prepared by tableting the following formulations according to a conventional method. The tablets thus prepared were stored in polyethylene bags at 40°C for one week.

**Table 1**

| | Examples of formulations | | | | | |
|---|---|---|---|---|---|---|
| | No. 1 | No. 2 | No. 3 | No. 4 | No. 5 | No. 6 |
| Powder sugar | 93.0 | 93.0 | 93.0 | 93.0 | 93.9 | 93.0 |
| Sucrose fatty acid ester | 1.5 | 1.5 | 1.5 | 1.5 | 1.5 | 1.5 |
| Powdered peppermint flavor | 0.5 | 0.5 | 0.5 | 0.5 | 0.5 | 0.5 |
| Product 1 of the invention | 5.0 | - | - | - | - | - |
| Product 2 of the invention | - | 5.0 | - | - | - | - |
| Product 4 as comparison | - | - | 5.0 | - | - | - |
| Reference Product 1 | - | - | - | 5.0 | - | - |
| Product 5 as comparison | - | - | - | - | 5.0 | - |
| Reference Product 2 | - | - | - | - | - | 5.0 |
| Total (g) | 100.0 | 100.0 | 100.0 | 100.0 | 100.0 | 100.0 |

Surface of the above tablets was microscopically observed, while one tablet (1.5 g) was dissolved in 30 g of water and the emulsified particles were microscopically observed. As a result, no oil exudation over the tablet surface was observed in the formulations No. 1 and No. 2 in which Products 1 and 2 of the invention were incorporated respectively, and microscopic observation showed that the emulsified particles after being dispersed in water had a maximum size of around 3 µ and any disintegration of emulsified particles caused by compressing was not observed. In the formulation No. 3 or No. 5 in which Comparison Product 4 or 5 was incorporated, slight oil exudation over the surface of tablets was observed and partial disintegration of the emulsified particles was also observed, but each formulation was sufficiently applicable to practical use. On the other hand, in the formulation No. 4 or No. 6 in which Reference Product 1 or 2 was incorporated, partial oil exudation over the surface of tablets was observed and coalesced emulsified particles were observed by microscopic observation after being dispersed in water. After storing the tablets at 40°C for one week, organoleptic tests for their flavor were carried out by ten special panelists. It was judged that the formulations No. 1 and No. 2 in which Products 1 and 2 of the invention were respectively incorporated smelt of no deterioration odor during storage. On the other hand, it was judged by all the panelists that the formulation No. 4 or No. 6 in which Reference Product 1 or 2 was respectively incorporated smelt of noticeable deterioration odor during storage. When the formulations were evaluated prior to tableting according to the same method as described above, no significant difference was observed in the formulations No. 1 to No. 6. It may be considered from the above results that deterioration odor during storage could be caused by disintegration of emulsified particles in the course of tableting.

It is demonstrated from the foregoing that powders coated with the chemically modified starch and vegetable gums show less disintegration in tableting and excellent tableting property and thus are extremely useful.

### INDUSTRIAL APPLICABILITY

According to the present invention, there is provided emulsified powders of an oil-soluble substance which show less disintegration of emulsified particles caused by compressing impact. The tableted product of the emulsified powders of the oil-soluble substance is easy in handling, weighing, ingesting and others and then can be utilized over a wide range of technical fields such as foods and drinks, medicaments or the like.

## Claims

1. A process for the preparation of emulsified powders which comprises emulsifying an oil-soluble substance, a chemically modified starch and a vegetable gum in water and drying the emulsion thus formed.

2. The process for the preparation of emulsified powders as claimed in claim 1, wherein said oil-soluble substance is an edible fat.

3. The process for the preparation of emulsified powders as claimed in claim 2, wherein said edible fat is a medicament or a health food.

4. The process for the preparation of emulsified powders as claimed in claim 2, wherein said edible fat is vitamin E or docosahexaenoic acid.

5. The process for the preparation of emulsified powders as claimed in any of claims 1 to 4, wherein said chemically modified starch is an ester of a starch with an alkenyl succinate.

6. The process for the preparation of emulsified powders as claimed in claim 5, wherein said alkenyl succinate is octenyl succinate.

7. The process for the preparation of emulsified powders as claimed in claim 5, wherein said chemically modified starch is octenyl succinate starch or sodium octenyl succinate starch.

8. The process for the preparation of emulsified powders as claimed in any of claims 1 to 7, wherein said vegetable gum is gum arabic.

## Patentansprüche

1. Verfahren zur Herstellung von emulglerten Pulvern, das umfasst: Emulgieren einer öllöslichen Substanz, einer chemisch modifizierten Stärke und eines pflanzlichen Gums In Wasser und Trocknen der so gebildeten Emulsion.

2. Verfahren zur Herstellung von emulgierten Pulvern nach Anspruch 1, worin die genannte öllösliche Substanz ein Speisefett ist.

3. Verfahren zur Herstellung von emulgierten Pulvern nach Anspruch 2, worin das genannte Speisefett ein Medikament oder Gesundheitsprodukt ist.

4. Verfahren zur Herstellung von emulgierten Pulvern nach Anspruch 2, worin das genannte Speisefett Vitamin E oder Docosahexaensäure ist.

5. Verfahren zur Herstellung von emulgierten Pulvern nach irgend einem der Ansprüche 1 bis 4, worin die genannte chemisch modifizierte Stärke ein Ester von Stärke mit einem Alkenylsuccinat ist.

6. Verfahren zur Herstellung von emulgierten Pulvern nach Anspruch 5, worin das genannte Alkenylsuccinat Octenylsuccinat ist.

7. Verfahren zur Herstellung von emulgierten Pulvern nach Anspruch 5, worin die genannte chemisch modifizierte Stärke Octenylsuccinat-Stärke oder Natriumoctenylsuccinat-Stärke ist.

8. Verfahren zur Herstellung von emulgierten Pulvern nach irgend einem der Ansprüche 1 bis 7, worin das pflanzliche Gum Gummiarabikum ist.

## Revendications

1. Procédé pour la préparation de poudres émulsionnées qui comprend les étapes consistant à émulsionner une substance liposoluble, un amidon. modifié chimiquement et une gomme végétale dans l'eau et à sécher l'émulsion ainsi formée.

2. Procédé pour la préparation des poudres émulsionnées selon la revendication 1, dans lequel ladite substance liposoluble est une matière grasse alimentaire.

3. Procédé pour la préparation des poudres émulsionnées selon la revendication 2, dans laquelle ladite matière grasse alimentaire est un médicament ou un aliment naturel.

4. Procédé pour la préparation de poudres émulsionnées selon la revendication 2, dans lequel ladite matière grasse alimentaire est la vitamine E ou l'acide docosahexanoique.

5. Procédé pour la préparation de poudres émulsionnées selon l'une quelconque des revendications 1 à 4, dans lequel ledit amidon modifié chimiquement est un ester d'un amidon avec un succinate d'alkényle.

6. Procédé pour la préparation de poudres émulsionnées selon la revendication 5, dans lequel ledit alcényle succinate est le succinate d'octényle.

7. Procédé pour la préparation de poudres émulsionnées selon la revendication 5, dans lequel ledit amidon modifié chimiquement est le succinate d'octényle amidon ou le sodium octényle succinate d'amidon.

8. Procédé pour la préparation de poudres émulsionnées selon l'une quelconque des revendications 1 à 7, dans lequel ladite gomme végétale est la gomme arabique.
